# EUROPEAN PATENT SPECIFICATION

(11) **EP 0 413 675 B1**
(45) Date of publication and mention of the grant of the patent: **03.05.1995**
(21) Application number: 90870127.9
(22) Date of filing: 13.08.1990
(51) Int. Cl.: C07H 13/04

(54) **Method for producing synthetic N-linked glycoconjugates**
Verfahren zur Herstellung von künstlichen N-verbundenen Glycoconjugaten
Procédé de production de glycoconjugates, N-liés, synthétiques

(30) Priority: 16.08.1989 US 394691
(43) Date of publication of application: 20.02.1991
(73) Proprietor: OXFORD GLYCOSYSTEMS LTD., Abingdon, Oxon OX14 1RG (GB)
(72) Inventor: Manger, Ian David, South Parks Road, Oxford, 0X1 3QU (GB); Dwek, Raymond Allen, South Parks Road, Oxford, 0X1 3QU (GB); Rademacher, Thomas William, South Parks Road, Oxford, 0X1 3QU (GB)
(74) Representative: Perry, Robert Edward

(56) References cited:
- WO-A-88/04323
- CARBOHYDRATE RESEARCH, vol. 80, 1980, pages 99-115, Amsterdam, NL; B. PAUL etal.: "Synthesis and biological activity of some 1-N-substituted 2-acetamido-2-deoxy-beta-D-glycopyranosylamine derivatives and related analogs"
- IDEM
- CARBOHYDRATE RESEARCH, vol. 146, 1986, pages C1-C5, Amsterdam, NL; L.M. KIKHOSHERSTOV et al.:"A new simple synthesis of amino sugar-beta-D-glycosylamines"
- IDEM
- CARBOHYDRATE RESEARCH, vol. 19, 1971, pages 231-241, Amsterdam, NL; D.E. COWLEYet al.: "Synthesis of some aminoacyl derivatives of 2-acetamido-2-deoxy-beta-D-glucopyranosylamine and of 2-amino-1-N-(4-L-aspartyl)-2-deoxy-beta-D-glucopyranosylamine"

## Description

### Background of the Invention

This invention relates to a method for the derivatization of oligosaccharides to form synthetic N-linked glycoconjugates under conditions that maintain the β-anomeric configuration.

In general, carbohydrates are attached to various conjugates (e.g., proteins and lipids) by either N(nitrogen)-glycosidic or O(oxygen)-glycosidic linkages. Most animal glycoproteins contain oligosaccharides that are linked to a polypeptide backbone by a N-glycosidic linkage between N-acetyl-glucosamine (GlcNAc) and asparagine (Asn). The nitrogen glycosidic linkage between the reducing terminal monosaccharide (pyranose form) and asparagine in glycoproteins is in the β-anomeric configuration as shown by Formula I, below: 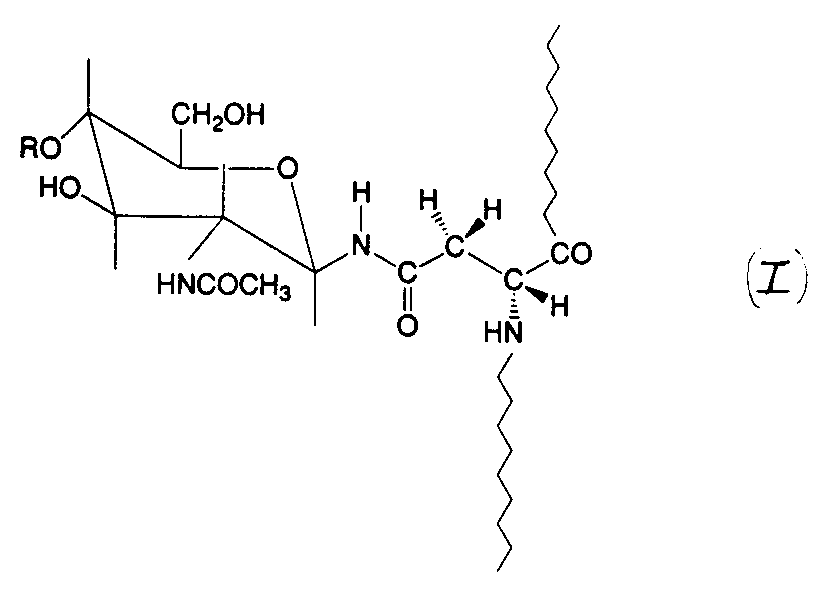
Oligosaccharides with a free reducing terminus can be isolated from a variety of plant and animal sources. In addition, oligosaccharides can be released from glycoproteins by chemical or enzymatic methods. These saccharides also have a reducing terminal monosaccharide residue, typically GlcNAc or GalNAc (N-acetyl-galactosamine).

Derivatives of these oligosaccharides are useful in basic research activities concerning the function of the carbohydrate moieties of naturally occurring glycoconjugates, in clinical research and diagnostic medicine and in clinical pharmacology and therapeutics. The following list illustrates these useful derivatives.
1) Biotin conjugates of oligosaccharides.
2) Fluorescent conjugates of oligosaccharides.
3) Lipid conjugates of oligosaccharides.
4) Peptide conjugates of oligosaccharides.
5) Amino-acid conjugates of oligosaccharides.
6) Immobilized oligosaccharide to solid support (e.g. agarose gel columns, silicon chips, Petri dishes etc.).
7) Drug conjugates of oligosaccharides.
8) Chromophore conjugates of oligosaccharides.
9) 1-N-Protected glycosylamine derivatives, e.g. with carbobenzoxy (CBZ) or 9-fluorenylmethoxycarbonyl (FMOC) protecting groups.

Glycosylamines also are valuable intermediates in the synthesis of N-nucleosides, glycosylthioureas and glycosylamino heterocycles of biological and pharmaceutical interest. See, e.g., Carbohydr. Res. 188, 35-44 (1989), and references cited therein.

It would be desirable to make these oligosaccharide derivatives such that the linkage between the asparagine and the reducing terminal GlcNAc (i.e. GlcNAc→Asn) which occurs in glycoproteins as in formula I is preserved. That is, it would be desirable to maintain the β-anomeric configuration, pyranose form and carbonyl and methylene components of the asparagine. Formula II illustrates the nature of the GlcNAc→Asn linkage in glycoproteins; Formula III shows the chemical form of illustrative derivatives which thus would preserve the characteristics of the GlcNAc→Asn linkage. 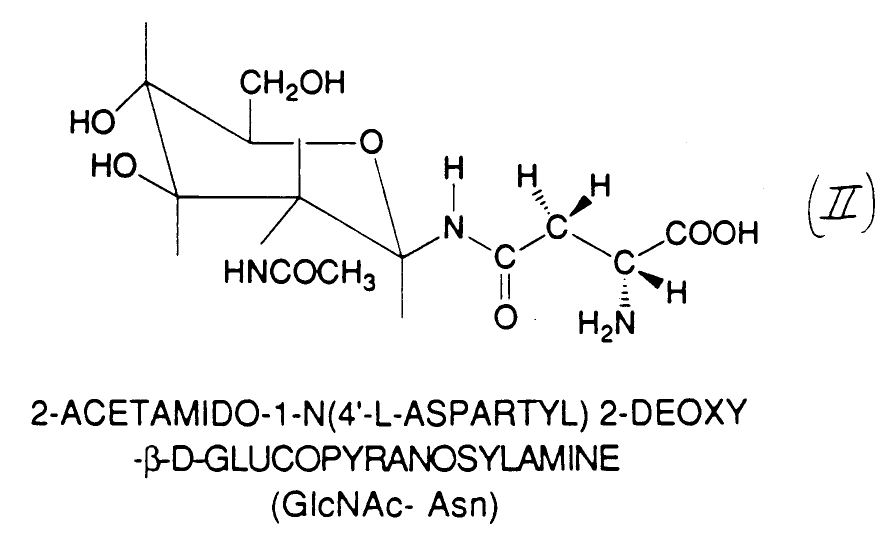 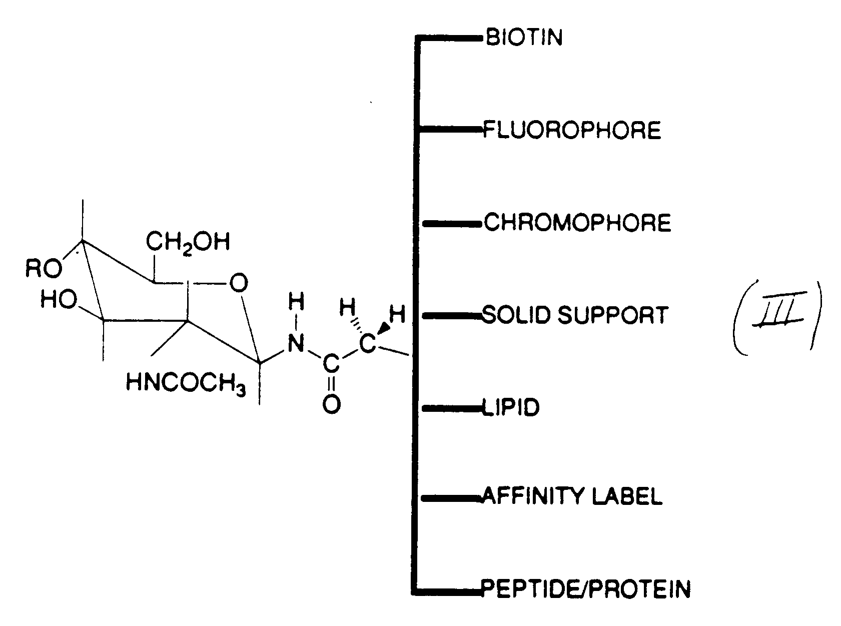
A number of published methods for derivatizing oligosaccharides are available but they do not preserve all the aforesaid desired characteristics of the GlcNAc→Asn linkage.

One prior method of oligosaccharide derivatization involves reductive amination as described, for example, by Stowell and Lee, Adv. Carbohydr. Chem. and Biochem. 37, 225-279 (1980), especially pg. 245. However, the described techniques do not preserve the pyranose form and the anomeric centre of the reducing terminal monosaccharide, nor the carbonyl and methylene groups of asparagine as can be seen from the following two illustrative reaction schemes: 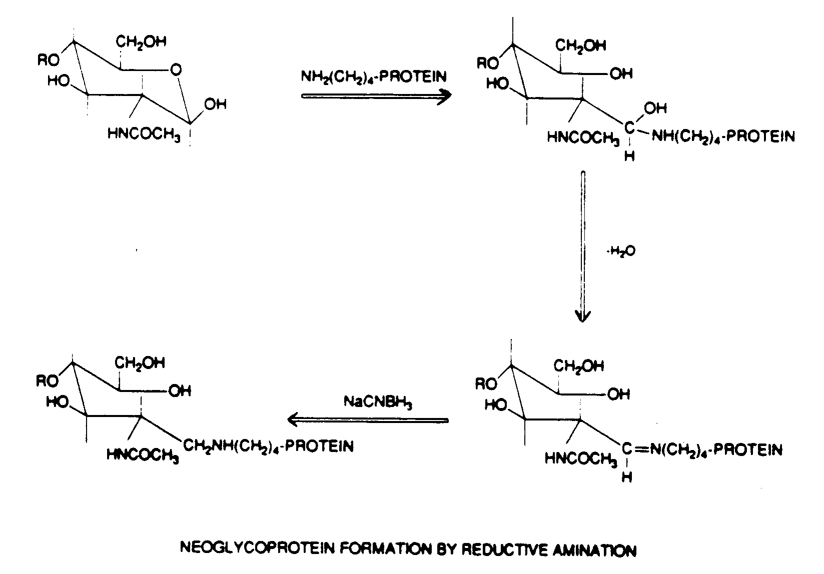 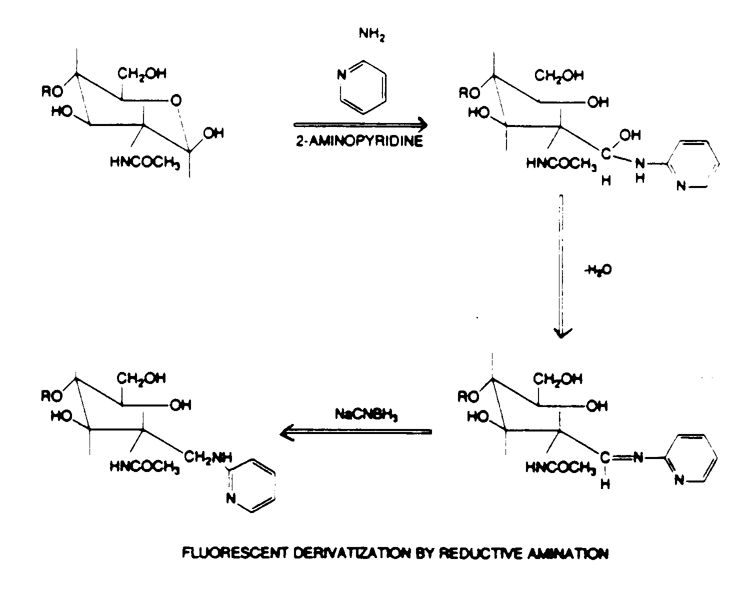
Another prior method of oligosaccharide derivatization involves formation of glycoconjugates by direct derivatization of glycosylamines as illustrated in PCT Inter. Pat. Appln. WO 88/04323, published June 16, 1988. Although these techniques preserve the pyranose form of the reducing GlcNAc, they do not necessarily preserve the β-anomeric configuration (a mixture of products is obtained) nor the carbonyl and methylene group of the asparagine as seen from the following illustrative product formula IV. Moreover, the methodology is applicable only to N-linked oligosaccharides attached to glycoproteins and, thereby, is of limited use.

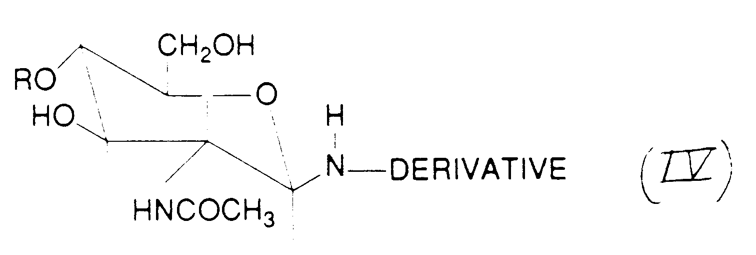

A further prior art method of oligosaccharide derivatization has been described by B. Paul et al. (Carbohydrate Research, 80, 99-115 (1980) ). B. Paul et al. disclose the synthesis and biological activity of some 1-N-substituted 2-acetamido-2-deoxy-β-D-glycopyranosylamine derivatives and related analogs. Starting with 2-acetamido-3,4,6-tri-O-acetyl-2-deoxy-β-D-glucopyranosylamine, obtained by catalytic reduction of 2-acetamido-3,4,6-tri-O-acetyl-2-deoxy-β-D-glucopyranosylazide, several analogs were synthesized by acetylation of the 1-amino group. The analogs have either highly polar groups (e.g. the trifluoromethanesulfonyl group) or potential alkylating groups, such as the haloacetyl derivatives.

### FORMATION OF GLYCOCONJUGATES BY DIRECT DERIVATIZATION OF GLYCOSYLAMINES

### Brief Description of the Invention

In accordance with the present invention, a novel method is provided for the derivatization of oligosaccharides to form synthetic N-linked glycoconjugates under conditions that maintain the β-anomeric configuration. It has been found that the β-anomeric configuration can thus be substantially preserved by converting a glycosylamine derivative of said oligosaccharide to a haloacetylated derivative as an intermediate compound prior to formation of the desired N-linked glycoconjugate. Preferably, the haloacetylated derivative is a chloroacetylated derivative. Conversion of the glycosylamine to a haloacetylated derivative can be carried out by reaction with a reagent capable of donating a haloacetyl function to the glycosylamine. In accordance with the invention, any reducing monosaccharide or polysaccharide with a reducing monosaccharide can be thus derivatized.

In accordance with the invention, the oligosaccharide derivatization is employed in an overall three step method as follows:
1) Synthesis of a glycosylamine derivative of the oligosaccharide such that the pyranose form and the β-configuration of the reducing terminal monosaccharide (usually GlcNAc) is preserved.
2) Synthesis of a haloacetylated derivative of the glycosylamine. This synthetic step must not involve mutarotation of the β-configuration of the glycosylamine.
3) Conversion of the haloacetylated derivative to either a synthetically useful intermediate or direct derivatization of the haloacetylated glycosylamine with a conjugate.

In the method of the invention, the haloacetylation is preferably carried out by reaction of the glycosylamine with an excess of chloroacetic anhydride (also referred to as sym. dichloroacetic anhydride). In the case of the bromo- and iodo-derivatives, the NHS-ester of the acid can be used, e.g. ICH₂COONHS. Preferably, a molar excess of at least about 5-fold, e.g. 5- to 10- fold, of the N-acetylation reagent is thus used. Use of the chloracetic anhydride surprisingly results in an intermediate which remains about 98-99% in the β-anomeric configuration compared to prior art methods, e.g. dansyl chloride, which result in mixtures with significant amounts of α-anomeric product. Although chloroacetic anhydride is a known reagent for the N-acetylation of amino acids, and ammonolysis of halogen fatty acids to prepare α-amino acids is known [Cheronis and Spitzmueller, J. Amer. Chem. Soc. 61, 349-375 (1941)], its high N-specific reactivity with the glycosylamine and the retention of the β-anomeric configuration in the formation of N-linked oligosaccharides as described herein were unexpected.

In the overall three step method, the initial glycosylation of the oligosaccharide is preferably carried out by incubation of the oligosaccharide in saturated ammonium bicarbonate at moderate alkaline pH of about 8-8.5, and preferably at about pH 8.3. Glycosylation under these conditions results in a glycosylamine in the β-anomeric configuration.

Following the haloacetylation reaction as described above, the desired haloacetylated glycosylamine intermediate can be used to synthesize a variety of N-glycyl-glycosylamines or N-linked glycoconjugates as described hereinbefore. For example, a fluorescent conjugate of the oligosaccharide can be made by reaction of the N-glycyl-glycosylamine intermediate with a fluorophore such as a fluorescein or rhodamine derivative.

Use of the haloacetyl donating reagent to form the intermediate haloacetylated derivative of the glycosylamine prior to formation of the glycoconjugate is critical to the invention. It has been found that direct derivatization of the glycosylamine such as by use of a trapping agent such as acid chlorides, acid anhydrides and other active acyl compounds as disclosed in PCT Inter. Appln. WO 88/04323 is not a practical method to produce glycoconjugates in high yield in the β-anomeric form. Also, acid chlorides will react with the hydroxy group of the sugar, i.e. it is not an N-specific derivatization reagent, and acetic anhydride forms a synthetically useless derivative of the glycosylamine.

### Detailed Description of the Invention

While the specification concludes with claims particularly pointing out and distinctly claiming the subject matter regarded as forming the present invention, it is believed that the invention will be better understood from the following detailed description of preferred embodiments taken in conjunction with the appended drawings, in which:
FIG. 1 is a graphical representation which shows the pH dependence of mutarotation and hydrolysis of glycosylamines.
FIG. 2 is a graphical representation which shows the removal of ammonium bicarbonate from oligosaccharides with time during lyophilization.
FIG. 3 is a graphical representation which shows the rate of formation of glycosylamine during incubation of oligosaccharide in saturated ammonium bicarbonate in one embodiment of the invention.
FIG. 4 is a graphical representation which shows the HPLC elution profile of product obtained by chloroacetylation of the glycosylamine of FIG. 3.
FIG. 5 is a graphical representation which shows the HPLC elution profile of products from the ammonolysis of the chloroacetylated glycosamine of FIG. 4.
FIG. 6 is a graphical representation which shows the completion of a second ammonolysis reaction as in FIG. 5.
FIG. 7 is a graphical representation which shows the reverse phase HPLC elution profile of a fluorescein derivative of the N-glycyl-glycosylamine derivative of N-acetylglucosamine in another embodiment of the invention.

It will be appreciated that the oligosaccharides used in the method of the invention can be isolated or derived from a variety of plant and animal materials such as, for example:
(1) Purified glycoproteins and glycohormones;
(2) Whole serum and its fractions;
(3) Biological secretions such as, for example, urine, milk, meconium, mucus, colostrum and the like substances;
(4) Whole organs, for example, kidneys, liver, heart, spleen, pancreas, lung;
(5) Plant stem and leaf extracts;
(6) Seed material;
(7) Lectins; and
(8) Emulsins.

Release of oligosaccharides from such plant and animal material by chemical means such as hydrazinolysis is described in U.S. Patents 4,719,294 and 4,736,022 and by Takasaki et al., Meth. Enzymol. 83, 263-268 (1982).

Release of oligosaccharides by enzymatic methods is illustrated by the use of N-glycanase as described by Hirani et al., Anal. Biochem. 162, 485-492 (1987).

The derivatization of the oligosaccharides to form, synthetic N-linked glycoconjugates under conditions that maintain the β-anomeric configuration is illustrated hereinafter in detail by the overall three step method.

### STEP 1. FORMATION OF GLYCOSYLAMINES

Prior to the present invention, no adequate general method existed for the synthesis of glycosylamines.

The following methods have previously been described.
1. A method for producing glycosylamines via a glycosyl azide [Garg and Jeanloz, Adv. Carbohyd. Chem. and Biochem. 43, 135-139 (1985); Cowley et al., Carbohydr. Res. 19, 231-241 (1971); and Nakabayashi et al., Ibid. 174, 279-289 (1988)].
2. A method for producing glycosylamines using methanolic ammonia [Frush and Isbell, J. Org. Chem. 23, 1309 (1958); Frush and Isbell, J. Res. Natl. Bur. Stds. 47 (4), 239-247 (1951)]. This method is not applicable to larger structures owing to their insolubility in this solvent system, and the susceptibility of reducing terminal N-acetylglucosamine residues to undergo base catalyzed epimerization at C2, or to β-elimination of 1-3 linked core fucose.
3. A one step condensation of a saccharide and ammonia using saturated ammonium bicarbonate [Likhosherstov et al., Carbohydr. Res. 146, C1-C5 (1986)].
4. Enzymatic methods whereby glycoproteins are reacted with a β-aspartyl glycosylamine amidohydrolase (PCT Inter. Pat. Appln. WO 88/04323).

A modification of the general ammonium bicarbonate procedure is used herein. The formation of glycosylamines by the condensation of monosaccharides and ammonia has been extensively studied by Frush and Isbell as noted above. The reaction is thought to proceed via the acyclic ammonium ion (Schiff adduct) followed by recyclization to give the glycosylamine. The method of Likhosherstov et al. uses ammonium bicarbonate as the ammonia source rather than the methanolic ammonia used by Isbell and Frush. This method has the advantages that larger oligosaccharides may be soluble in this aqueous system. While ammonium salts may increase the formation of bisglycosylamines by the splitting out of ammonia between two glycosylamine molecules, low concentrations of sugar minimize this side reaction.

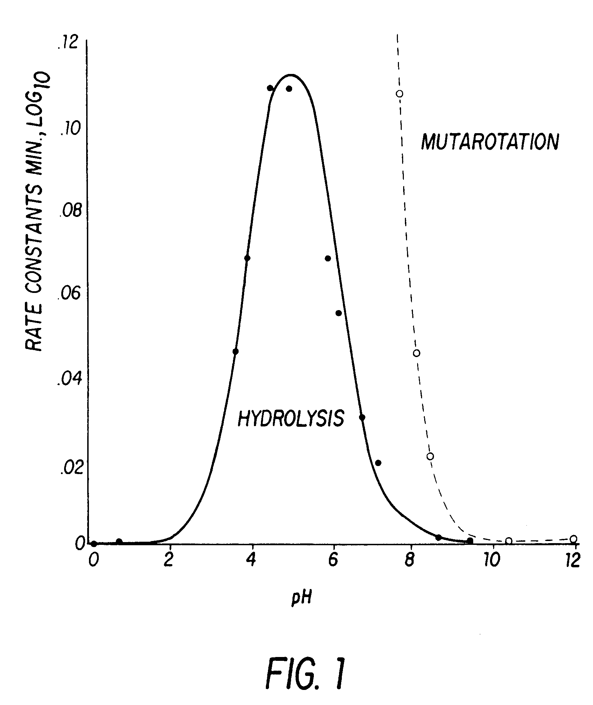

Glycosylamines undergo rapid ring opening rearrangements and the outcome of these is strongly dependent on the pH. Above pH 8.0 they mutarotate rapidly with the equilibrium in favor of the β-form as shown by Isbell and Frush, J. Org. Chem. 23, 309 (1958) (see Fig. 1). Glycosylamines are readily hydrolyzed at moderately acidic pH (∼4.5) (see Fig. 1). As a consequence of these chemical properties, any work-up procedure and subsequent synthetic reactions must be adequately buffered to balance general acid catalyzed hydrolysis (by a reverse of the reaction sequence shown in Fig. 1) and to maintain the amine as a reactive nucleophile (i.e. deprotonated).

The formation of glycosylamines and the yield obtained is therefore dependent on the use of moderate basic conditions which minimize side reactions of the starting sugar, and avoidance of acidic conditions which catalyze hydrolysis of the amine. Further, the possibility of base catalyzed epimerization at C2 and β-elinination occurring can be minimized if the reaction occurs at moderately alkaline pH of about 8-8.5 and preferably about 8.3.

A summary of the general ammonium bicarbonate method of Likhosherstov et al. is shown by the following reaction scheme:

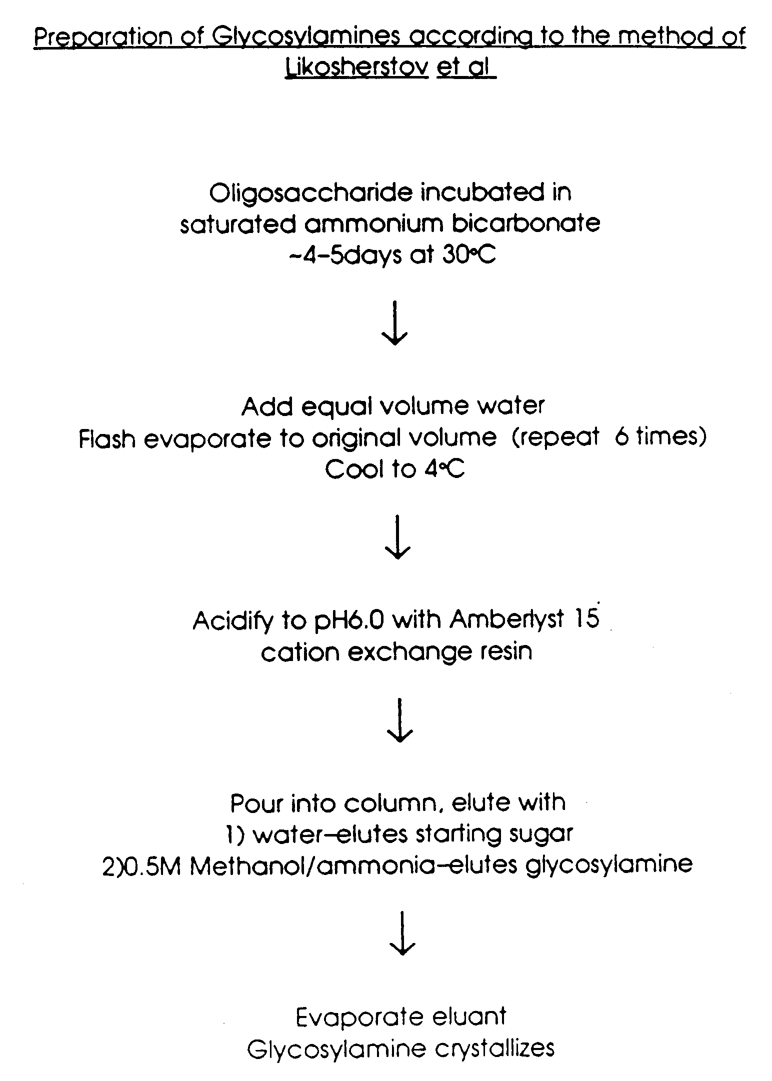

The authors claim that this method is effective in producing glycosylamines of simple mono-and disaccharides in 60% yield. However, the present inventors have not been able to duplicate their yields using the published procedure. The following observations were made.
1) The acidification of the reaction mixture must be very carefully controlled, since the hydrolysis of the glycosylamine is most rapid at moderately acidic pH (see Fig. 1). This step is difficult to control with a small oligosaccharide sample (analytic scale).
2) The Amberlyst exchange resin used is unstable in the methanolic ammonia eluant. Despite extensive washings the resin was solubilized and appeared as a discolorant. Further investigation of the properties of the Amerlyst resin (see Experiments below) also showed that it was not possible to quantitatively elute glycosylamines under the published conditions. Another resin, AG50 x 12(H⁺) was tested, but it also bound the glycosylamine strongly at acidic pH. The simple glycosylamine of N-acetylglucosamine could not be quantitatively eluted from columns of this resin.

### Experimental

Acidification of saturated ammonium bicarbonate was performed according to the method of Likkosherstov et al. Since hydrolysis is most rapid at mildly acidic pH (see above), this was carefully controlled.

For elution experiments, 200 »l (560 »Eq) of saturated ammonium bicarbonate containing ³H-GlcNAc was incubated for 4 days at 30°C and then applied to a column of 400 »l (600 »Eq) Amberlyst® resin which had previously been extensively washed with 10 column volumes 1M HCl, 10 column volumes of 0.5M MeOH/NH₃ and then with 20 column volumes of water. Unbound sugar (free N-acetylglucosamine) was eluted by 10 column volumes of water, evaporated to dryness and counted. Bound glycosylamine was eluted using various concentrations of ammonia/methanol as indicated in Table I. The eluants were dried and counted.

Table I shows the amount of bound monosaccharide eluted from the resin using these eluants. From these data it is evident that a large fraction of the amine remains strongly bound to the resin. The nature of this interaction is unknown.

**Table I**

| Elution of ³H-N-acetylglucosamine from Amberlyst 15 resin | | |
|---|---|---|
| Eluant | % Bound | % Free |
| Water | 83.5% | 15.5% |
| 0.5M | 71.4%* | |
| 1.0M | 50.8%* | |
| 2.0M | 39.9%* | |
| 4.0M | 36.0%* | |

| | | |
|---|---|---|
| *Percent of material not eluted by water | | |

These problems prompted the present inventors to develop other methods of isolation which can be generally applied to oligosaccharide glycosylamines (both analytic and preparative).

### Removal of ammonia during glycosylamine synthesis.

The complete removal of the ammonium bicarbonate from the mixture is a major problem. From the results above, quantitative recovery of the glycosylamine from ion-exchange resins is not believed to be possible. Desalting of larger glycosylamines may be simply achieved by using gel filtration chromatography eluted with water, but associated problems generally occur as follows:
1) hydrolysis of the glycosylamine in water.
2) oligosaccharides can interact with the column matrix giving rise to non-quantitative recoveries.

The present inventors found that significant amounts of the ammonium salts may be removed without hydrolysis of the glycosylamine, by the addition of methanol to approximately 90% by volume. This decreases the solubility of the salt from 220 mg/ml (20°C) to about 0.01 mg/ml. The precipitated salts can be filtered off and washed to recover any surface bound sugar. Remaining salts can be can be removed by lyophilization. In practice, it was found that small oligosaccharide samples can be lyophilized directly from the reaction mixture.

### Example 1

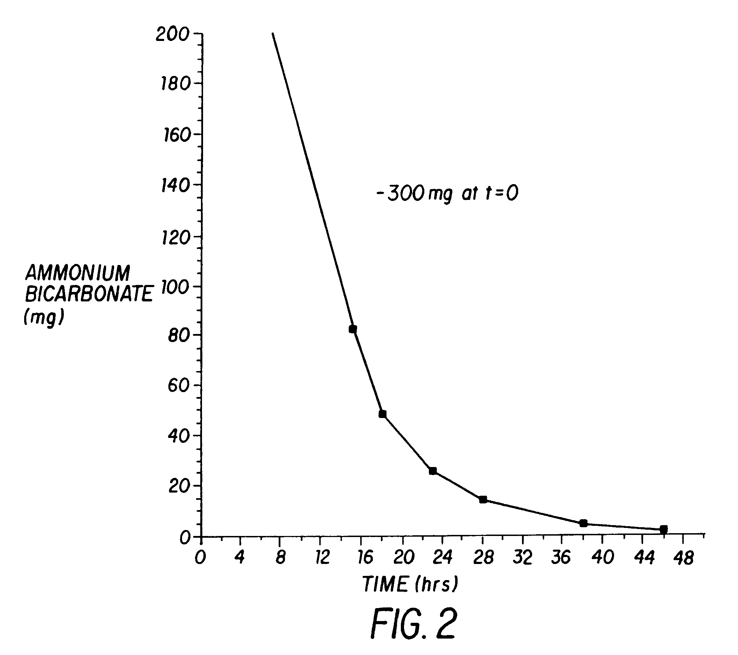

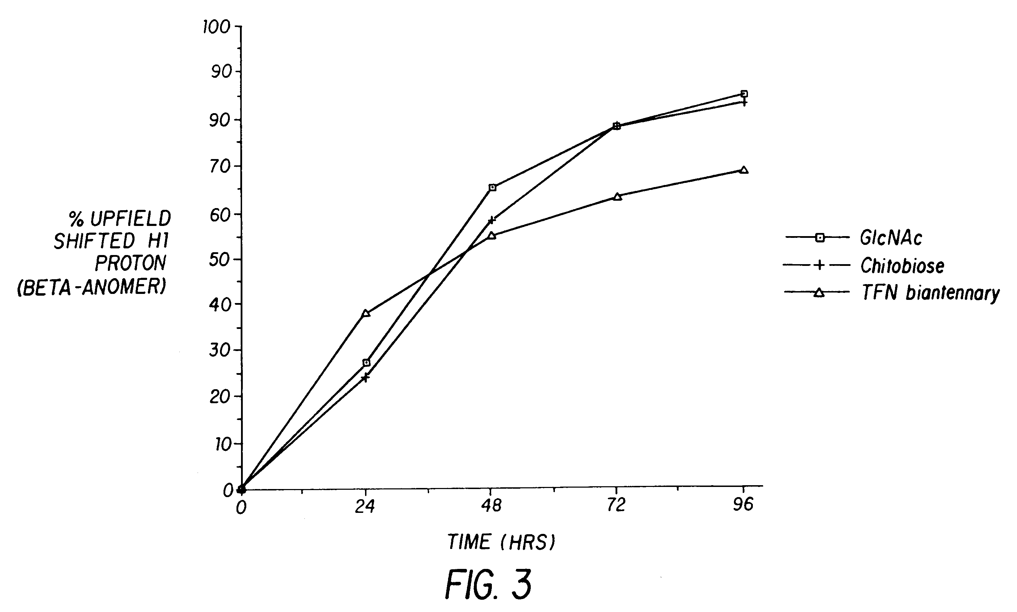

Samples of oligosaccharide in saturated ammonium bicarbonate (typically 50-100»l) were diluted to 1 ml and shell frozen in dry ice. These were then lyophilized at a chamber pressure of 10⁸ Pa (10³ bar). Figure 2 shows the removal of ammonium bicarbonate with time. In practice, the removal of ammonium bicarbonate can be accelerated by 6 hr repeat cycles of addition of water and lyophilization (data not shown). At the end of the lyophilization, samples are stored at -20°C in the presence of dessicant. Under these conditions they were found to be stable over at least a one month period. The rate of their formation as determined by ¹H-NMR Spectroscopy described below is shown in Fig. 3. Note that the glucosamines are in the pyranose form with an α/β anomer ratio of ∼1:24.

### ¹H-NMR spectra of glycosylamines

The formation of glycosylamine from the reducing sugar can be followed by ¹H-NMR spectroscopy. The condensation of sugar with ammonia is indicated by the collapse of the anomeric protons of the free sugar, and the appearance of a major resonance associated with the β-anomer of the glycosylamine, which for steric reasons (⁴C₁ configuration) is the more stable anomer. For N-acetylglucosamine the anomeric protons of the free sugar were found to resonate at 5.19ppm (α) and 4.70ppm (β), with associated J_{1,2} values of ∼3.5Hz and ∼8Hz, respectively. The glycosylamine has a major resonance at 4.15ppm, and from the J_{1,2} value of ∼8Hz, this can be identified as the β-anomer. Other minor components can also be identified, one of which is the α-anomer (4.39ppm, J_{1,2} ∼4.7Hz) (α/β ratio 1:24).

### Experimental

A sample of GlcNAc, GlcNAcβ1→4GlcNAc and Galβ1→4GlcNAcβ1→2Manα1→6[Galβ1→4GlcNAcβ1→2Manα1→3]Manβ1→ 4GlcNAcβ1→4GlcNAc was derivatized using ammonium bicarbonate, and at 24hr time points samples were withdrawn and lyophilized. These were then exchanged into D₂O (99.96 atom) and 1D-spectra obtained. Integrals of the anomeric region were taken, and by comparison with the methyl region the percentage of each species was obtained. The results obtained are shown in the Figure 3. These data suggest that condensation of the sugar with ammonia is complete after 3-4 days at room temperature.

### STEP. 2 HALOACETYLATION OF GLYCOSYLAMINES

Halocetylation was performed by reacting the glycosylamine with, for example, chloroacetic anhydride as illustrated by the following reaction scheme: 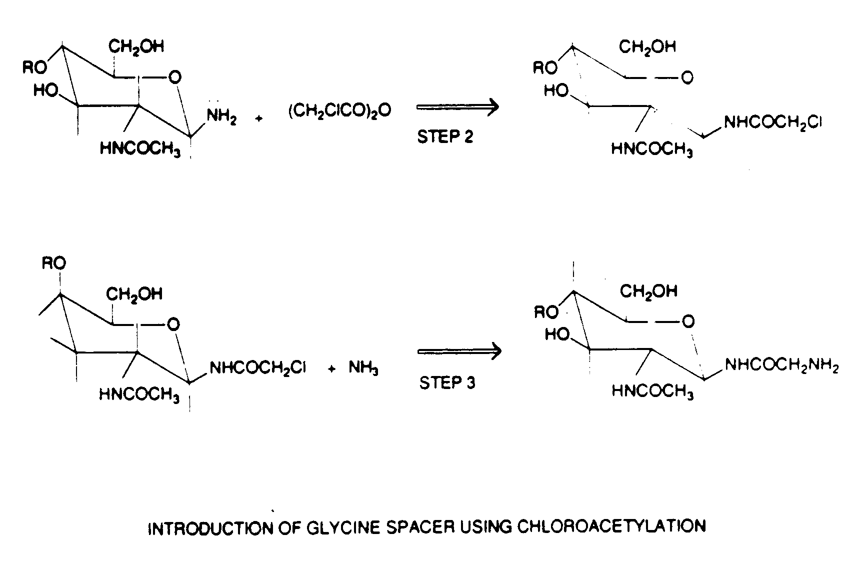
In the case of bromo- and iodo-derivatives the NHS-ester of the acid can be used as illustrated by the following reaction scheme using ICH₂COONHS:

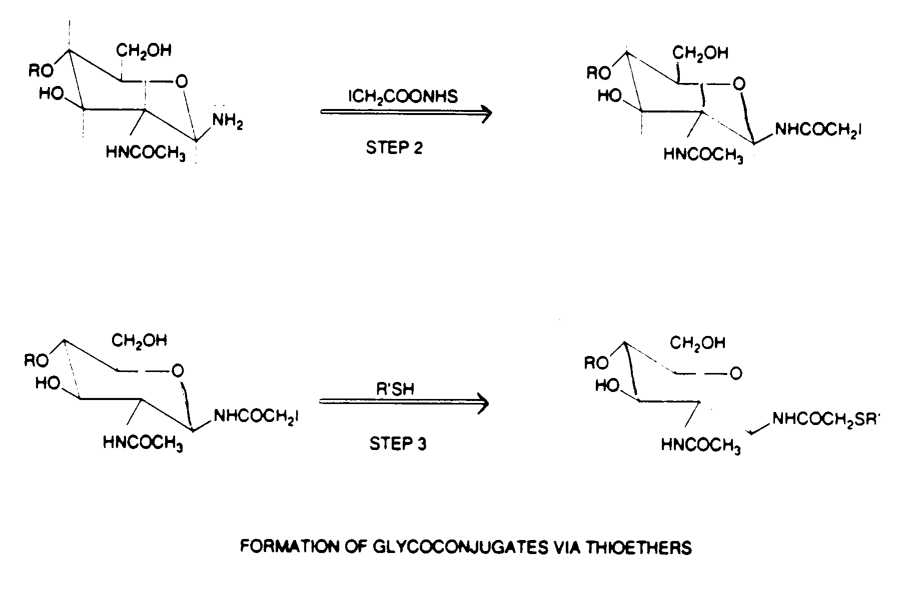

### Example 2

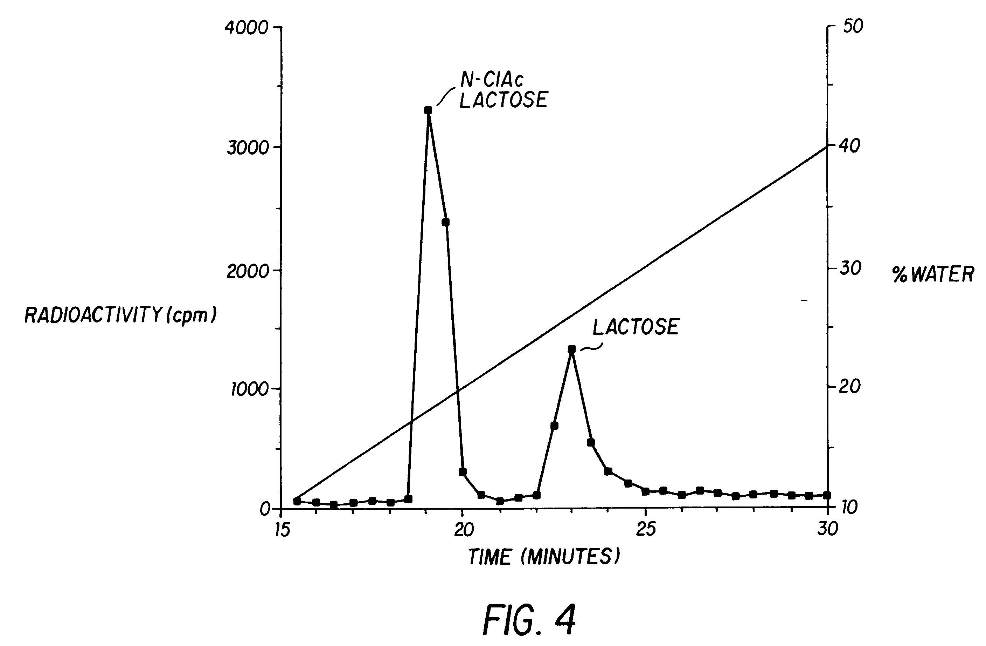

¹⁴C-lactosylamine 11.84 x 10⁶ Bq (0.32mCi/mmole) was made by condensation with ammonium bicarbonate. Following removal of the ammonium salts as described in Step 1, above, the sample was resuspended in 1.0M NaHCO₃ and chloroacetylated by the addition of a five-fold molar excess of sym. dichloroacetic-anhydride (Fluka Biochemicals). After a 2 hr. incubation at room temperature a second quantity of base and anhydride was added. After a further 6 hrs. the mixture was passaged over a mixed bed of AG50-X12(H⁺) and AG3-X4A(OH⁻) ion exchange resins. The eluant was collected and evaporated to dryness. Separation of the reaction products was performed using ion-supression amine absorption HPLC according to the method of Mellis and Baenzinger, Anal. Biochem. 134, 442 (1984) The reaction products were resuspended in 90% MeCN/10% water containing 50 mM triethylamine acetate (TEA) buffer, pH 5.5, and injected onto a Varian Micropak AX5 column equilibrated in the same buffer. Elution was performed using a 2%/min. gradient of TEA buffer following a 5 min. hold at the equilibration conditions. Radioactive products were detected using a Berthold LB503 HPLC radioactivity monitor. A typical profile is shown in Fig. 4. Radioactive fractions were pooled and dried.

The characterization of the 1-amino chloroacetyl-glycosylamine by NMR analysis as described below showed that the glycosylamine was still predominantly in the β-anomer configuration α/β (1:24) post haloacetylation. This contrasts with direct derivatization of the glycosylamine with, for example, dansyl chloride, which resulted in muta-rotation, as shown below.

### ¹H-NMR analysis of N-chloroacetyl and N-acetyl-glycyl-derivatives of 1-amino-N-acetyl glucosamine.

A sample of 1-amino-N-acetylglucosamine was N-chloroacetylated. At the end of the incubation period, the mixture was passed over a mixed bed of AG50-X12 and AG3-X4A ion exchange resins. The eluant was then evaporated to dryness under reduced pressure and lyophilized. It was then twice-exchanged into D20 and subjected to 1-D ¹H-NMR analysis. The spectrum obtained showed the presence of four distinct saccharide components in the anomeric region. These are the α- and β-forms of the free sugar and of N-chloroacetyl derivative. Inspection of the integrals for these four species gave an overall yield of ∼75% for formation of the chloroacetyl derivative, in a ratio of 24:1 in favor of the β-anomer.

The mixture was then subjected to ammonolysis at 50°C using an AG50x12-binding assay to follow the time course of the reaction. The glycyl-derivative was then purified on a short column of CM-Sepharose Fast Flow, eluted first with water and then with 0.5M ammonium carbonate. The material eluted by the salt was then pooled and the salt removed by evaporation. The mixture was then N-acetylated using acetic anhydride in saturated sodium bicarbonate and desalted using AG50/AG3 resins. The product was split into two for NMR analysis. One half was dried and redissolved in DMSO-d5 (Aldrich) and the other was exchanged into D20 and analyzed as described above.

A portion of the spectrum obtained in DMSO showed the downfield NH protons which are normally exchanged in D20. Two doublets at 7.84 and 7.88 ppm can be assigned as those of NH1 and NH2 on the basis of spin decoupling by irradiation at 4.55 ppm (triplet resonance of H1). The third resonance, a triplet at 8.09ppm, was assigned to the NH of the "glycine" acetamido function. Irradiation of this resonance causes perturbation of resonances between 3.40 and 3.60ppm which may assigned to the two methylene protons of the glycine space. Finally there are two well resolved methyl resonances at 1.70 and 1.80ppm.

### Reactivity of Glycosylamines (Dansyl and Fluorescein Derivatives)

The reaction between 1-amino-2-acetamido-1,2-dideoxy-D-glucopyranosyl-amine and dimethylaminonapthalenesulphonyl chloride (dansyl chloride) was performed by a modification of the method of Gray, Meth. Enz. XXV, 121, (1971). 10 »mole of the amine was dissolved in 200 »l of 0.5 M NaHCO₃. 200 »l of ethanol containing 24.5 mg dansyl chloride was then added with stirring and the reaction allowed to proceed at room temperature for 2 hrs. A brown solution containing some precipitated sodium bicarbonate was obtained. Following the addition of 100 »l of water to dissolve the precipitate, the products were separated using reverse phase chromatography on a Spherisorb S5ODS2 SP column (8.0 x 300 mm), using UV (258 nm) and fluorescence detection (exit. 336 nm-emiss. 536 nm). Column fractions were collected and 50 »l aliquots counted. Pooled fractions containing radioactivity were then evaporated to dryness, resuspended in 1ml water and counted to obtain the yield. Typical yields obtained by this method are 10-15% based on starting sugar.

The dansyl-aminosugar obtained by this method was lyophilized and then prepared for NMR analysis by twice exchanging into D₂O (99.96 atom) and finally redissolved in the same solvent. 1-D and 2-D analysis was performed using a Bruker 500Hz spectrometer. A 1D spectrum of the derivative showed the characteristic downfield aromatic resonances and well resolved anomeric, backbone and acetamidomethyl regions. This derivative showed a 1:4 ratio of the α and β forms respectively, compared with the 1:24 ratio observed in the unreacted glycosylamine (see above). There are at least two possible explanations for this observation, firstly that the dansyl derivative is capable of undergoing mutarotation via the ring open form, or secondly that the α-form reacts more rapidly than the β-form with dansyl chloride and that once formed the derivative is fixed as a particular anomer. Temperature and pH dependent studies favor the latter interpretation (data not shown).

The generally poor yields obtained for dansyl chloride were found to be typical of the reactions of glycosylamines. For example, similar yields were obtained with the isothiocyanate and NHS-esters of fluorescein. Acid chlorides such as dansyl chloride present an added difficulty in that there is the likelihood of O-acylation with these highly reactive species. Indeed, this was found to be the case when the kinetics of dansylation with various glycosylamine was studied (data not shown). The formation of O-dansyl derivatives represents a further route by which the acid chloride may be consumed.

It was therefore concluded that direct derivatization of glycosylamines results in α and β mixtures of the products and low yields were consistently found.

### STEP 3. SYNTHESIS OF N-GLYCYL-GLYCOSYLAMINES

The haloacetylated glycosylamine derivatives can be used in a number of synthetic strategies. For example, the halo-function can be replaced by a primary amine, such as in the synthesis of glycine using chloroacetic acid, or iodo-derivatives can be linked to thiols to form thioethers as shown by reaction schemes hereinbefore.

### Example 3

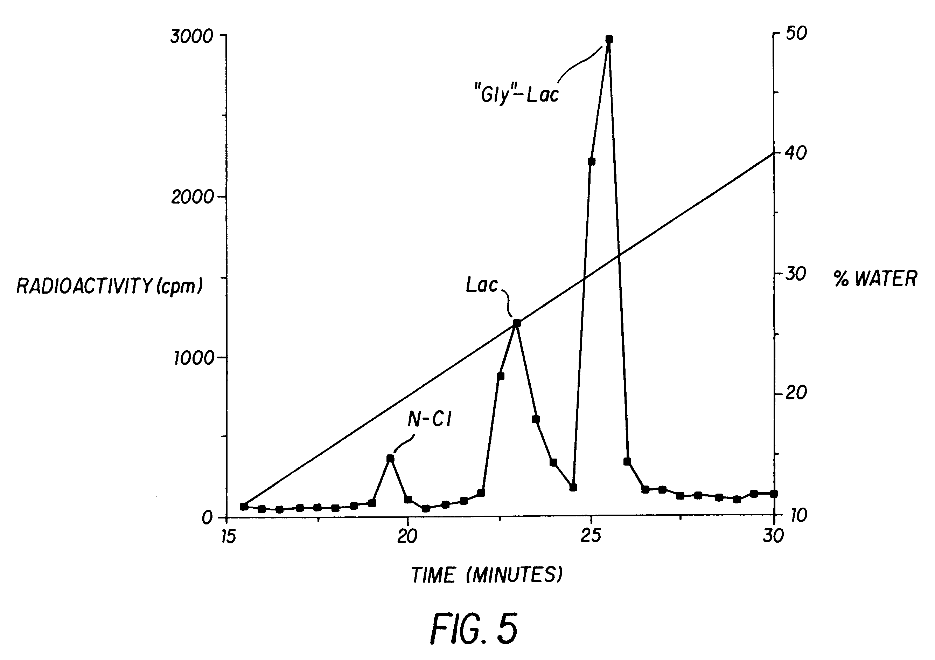

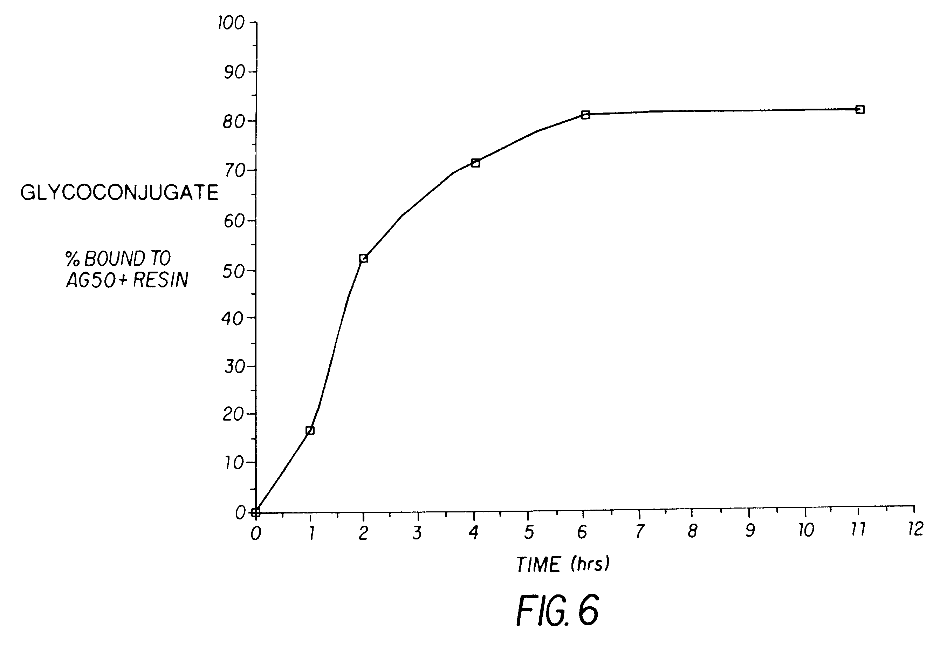

Ammonolysis of the haloacetate derivative was performed by incubation in saturated ammonium carbonate in a sealed tube (to prevent loss of ammonia by evaporation) at room temperature. The products were analyzed using the same HPLC method as above. In the reaction the chloroacetate derivative eluting at 18.8 minutes was converted slowly to a product eluting at ∼24 min. which was ninhydrin sensitive (see Fig. 5). The reaction was essentially complete after 96 hrs. at room temperature (see Fig. 5). A second ammonolysis reaction was carried out at 50°C and was found to be complete after overnight incubation. (see Fig. 6). An excess of ammonia over chloroacetyl function was found to be necessary to minimize the formation of secondary and higher amines.

### Synthesis of derivatives of N-glycyl-glycosylamine

### 1) Dansyl derivatives

The ammonolysis product (step 3 above) was purified and dansylated according to the method of Gray, Meth. Enzymol. XXV, 121 (1971), and the yield compared to that obtained by direct dansylation of lactosylamine (without formation of intermediate N-haloacetylated glycosylamine). The two reaction mixes were run on Silica 60 TLC using 7:1 MeCN/water containing 0.05% diaminobutane. The dansylated products were identified under UV light (366 nm) and these, and the origins (free lactose/N-glycl-lactosylamine) were eluted and counted. The results obtained are shown in Table II, below.

**Table II**

| Product | % of total |
|---|---|
| Dansyl-N-glycyl-lactosylamine | 69.5 |
| N-Glycyl-lactosylamine | 30.5 |
| N-Dansyl-lactosylamine | 10.1 |
| Free lactose | 89.9 |

### 2. Fluorescein derivatives

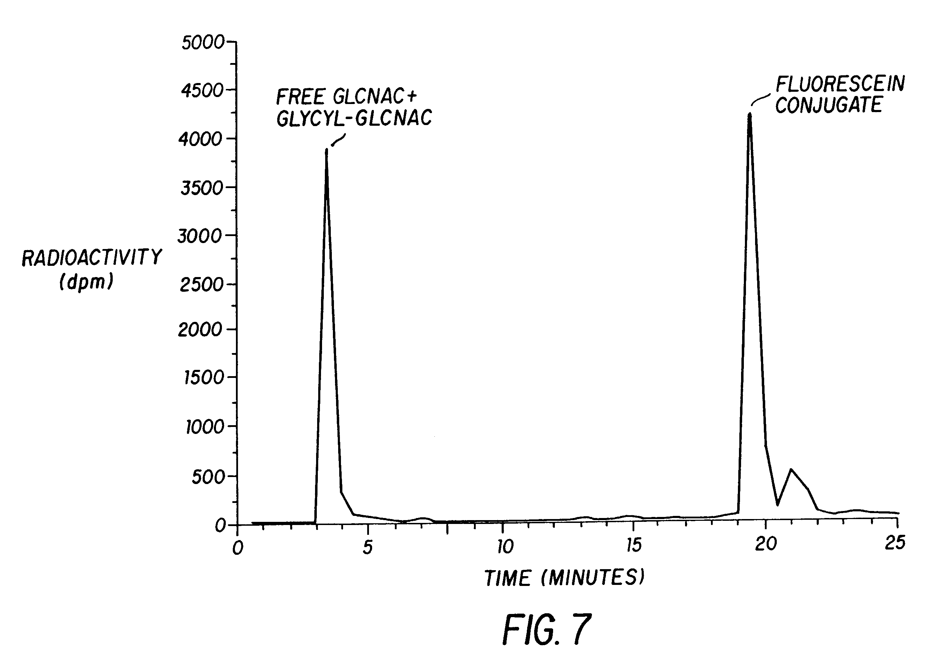

The N-glycyl-glycosylamine derivative of N-acetylglucosamine was dissolved in 100 »l of 0.1M sodium bicarbonate. To this was added 100 »l of DMF containing a 10-fold excess of 5(-6)-carboxyfluorescein-N-hydroxy-succinimidoester (Molecular Probes Inc., Eugene, Oregon). The reaction was allowed to proceed for 6 hrs at room temperature. The mixture was then dried, redissolved in water, acidified using glacial acetic acid and finally ether extracted to remove free fluorescein. The aqueous phase was then applied to reverse phase HPLC on a Spherisorb S50DS2 SP column (8.0 x 300 mm), using UV (258 nm) and fluorescence detection (exit. 336 nm-emisc. 536 nm). 50 »l aliquots of the fractions obtained were counted. A typical profile is shown in Fig. 7. Fractions containing free sugar and the fluorescein conjugate were then pooled, dried and counted to obtain the overall yield based on starting sugar shown in Table III below. Similar results were obtained with the derivatives of 5(-6)-carboxytetramethylrhodamine-N-hydroxysuccinimidoester (data not shown).

**Table III**

| Product | ³H dpm | % of total |
|---|---|---|
| Free GlcNAc | 3.19 E5 | 90.3 |
| Fluorescein-GlcNAc (direct) | 3.43 E4 | 9.7 |
| Free GlcNAc + GlycylGlcNAc | 1.24 E5 | 47.3 |
| Fluorescein-GlycylGlcNAc | 1.38 E5 | 52.6 |

The foregoing data indicate that haloacetylated-glycosylamines are an effective intermediate in the synthesis of β-N-linked saccharide conjugates. Since each of the steps proceeds practically to completion, derivatives in yields approaching 70-80% of total starting compound can be produced. 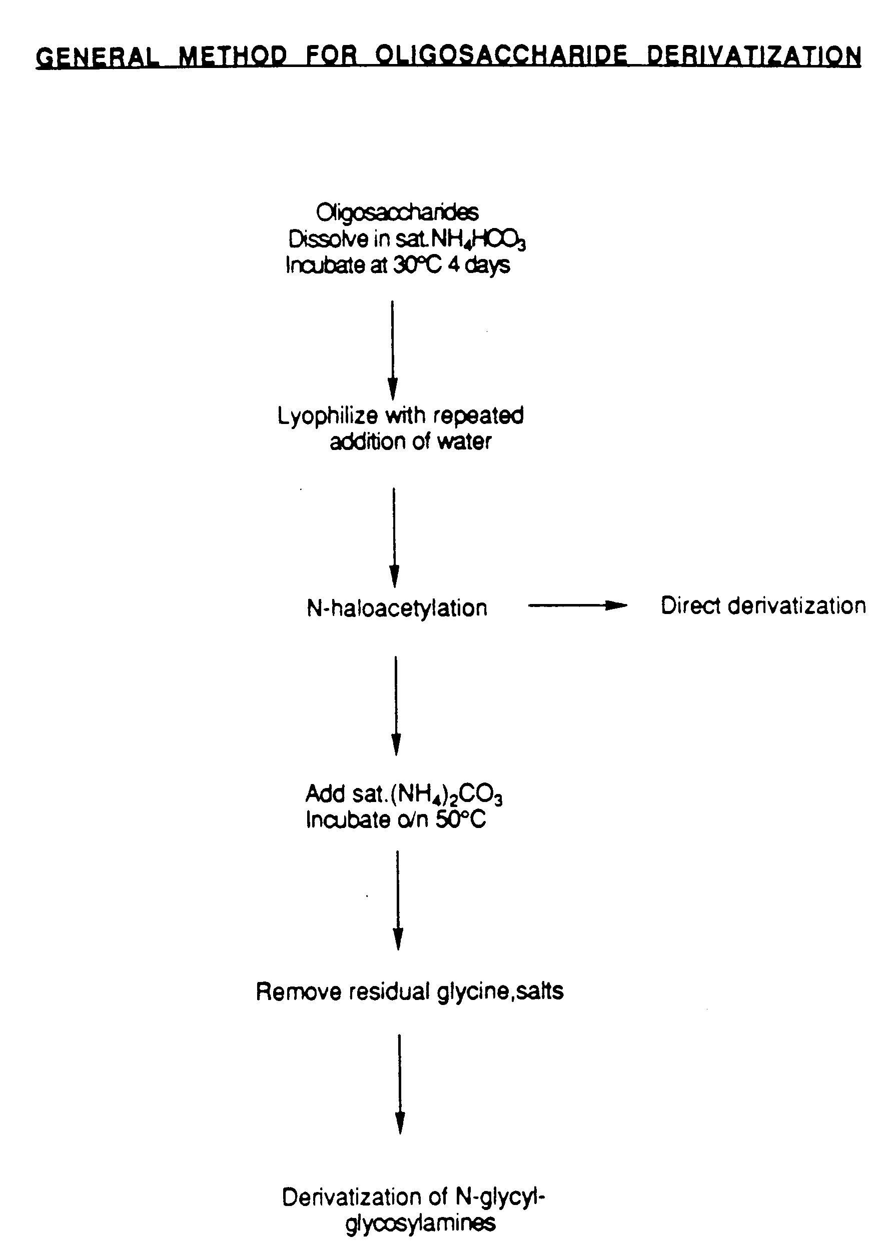
See the following reaction scheme for an additional example of the usefulness of these derivatives. It again should be pointed out that the structure of these derivatives includes the amide bond and methylene group found in the biological linkage between N-linked carbohydrates and asparagine in glycoproteins. The reactivity of haloacetylated glycosylamines and glycylglycosylamines with, e.g., thioglycolic acid and succinic anhydride, respectively, allow for the incorporation of a terminal carboxy group. This can be used to couple the oligosaccharide to glycopeptides or peptides or proteins by conventional EDC/NHS chemistry [1-ethyl-3-(3-dimethylaminopropyl)-carbodiimide/N-hydroxysuccinimide coupling reactions].

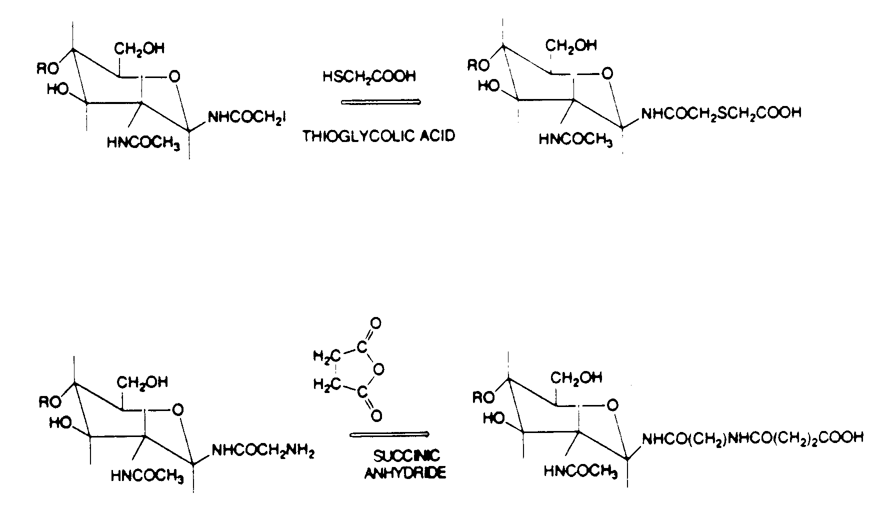

## Claims

1. A process for producing synthetic N-linked glycoconjugates of oligosaccharides under conditions to directly maintain the closed ring structure of the terminal monosaccharide of said oligosaccharides in the β-anomeric configuration comprising reacting an oligosaccharide in saturated ammonium bicarbonate at pH of from about 8 to about 8.5 to form a β-glycosylamine derivative of said oligosaccharide, haloacetylating said β-glycosylamine derivative in aqueous phase reaction to directly form the 1-N-haloacetamido derivative of said β-glycosylamine derivative without selective crystallization in organic solvent medium, converting said 1-N-haloacetamido derivative to a 1-N-glycyl-β-glycosylamine intermediate derivative by ammonolysis and thereafter reacting said 1-N-glycyl-β-glycosylamine intermediate derivative with a substrate capable of forming a 1-N-glycyl-β-glycosylamine linked glycoconjugate of said substrate and said 1-N-glycyl-β-glycosylamine derivative.

2. The method of Claim 1 in which the pH is about 8.3.

3. The method of Claim 1 or 2 in which said haloacetylated derivative is a chloroacetylated derivative thereof.

4. The method of Claim 3 in which chloroacetic anhydride is used to form the haloacetylated glycosylamine derivative.

5. The method of Claim 4 in which at least a 5-fold molar excess of chloroacetic anhydride is used to form the chloroacetylated derivative.

6. The method of Claim 3 in which the haloacetylated derivative is prepared by reaction of said glycosamine derivative of said oligosaccharide with ICH₂COONHS.

7. The method of any of claims 1 to 6 in which the glycyl-glycosylamine is reacted with a fluorophore to form a N-linked glycoconjugate substantially in the β-anomeric configuration.

8. The method of Claim 7 in which the fluorophore is a fluorescein derivative.

## Patentansprüche

1. Verfahren zur Herstellung synthetischer N-verbundener Glykokonjugate von Oligosacchariden unter Bedingungen, um die geschlossene Ringstruktur der Monosaccharid-Endgruppe der Oligosaccharide in der β-anomeren Konfiguration direkt aufrechtzuerhalten, welches umfaßt: Umsetzen eines Oligosaccharids in gesättigtem Ammoniumbicarbonat bei einem pH von etwa 8 bis etwa 8,5, wobei ein β-Glykosylamin-Derivat des Oligosaccharids gebildet wird, Halogenacetylieren des β-Glykosylamin-Derivats in einer Wasserphasenreaktion, wobei direkt das 1-N-Halogenacetamido-Derivat des β-Glykosylamin-Derivats ohne selektive Kristallisation in organischem Lösungsmittel-Medium gebildet wird, Überführen des 1-N-Halogenacetamido-Derivats in ein 1-N-Glycyl-β-glykosylamin-Zwischenderivat durch Ammonolyse, und danach Umsetzen des 1-N-Glycyl-β-glykosylamin-Zwischenderivats mit einem Substrat, das ein 1-N-Glycyl-β-glykosylamin-verbundenes Glykokonjugat des Substrats und des 1-N-Glycyl-β-glykosylamin-Derivats bilden kann.

2. Verfahren nach Anspruch 1, bei welchem der pH etwa 8,3 beträgt.

3. Verfahren nach Anspruch 1 oder 2, bei welchem das halogenacetylierte Derivat ein chloracetyliertes Derivat hievon ist.

4. Verfahren nach Anspruch 3, bei welchem Chloressigsäureanhydrid zur Bildung des halogenacetylierten Glykosylamin-Derivats verwendet wird.

5. Verfahren nach Anspruch 4, bei welchem ein zumindest 5-facher Molüberschuß von Chloressigsäureanhydrid zur Bildung des chloracetylierten Derivats verwendet wird.

6. Verfahren nach Anspruch 3, bei welchem das halogenacetylierte Derivat durch das Umsetzen des Glykosamin-Derivats des Oligosaccharids mit ICH₂COONHS hergestellt wird.

7. Verfahren nach einem der Ansprüche 1 bis 6, bei welchem das Glycylglykosylamin mit einem Fluorophoren umgesetzt wird, wobei ein N-verbundenes Glykokonjugat im wesentlichen in der β-anomeren Konfiguration gebildet wird.

8. Verfahren nach Anspruch 7, bei welchem der Fluorophore ein Fluorescin-Derivat ist.

## Revendications

1. Procédé de production de glycoconjugués d'oligosaccharides, synthétiques, à liaison par l'azote N, dans des conditions telles que la structure cyclique fermée du monosaccharide terminal desdits oligosaccharides est maintenue directement dans la configuration β-anomère, comprenant la réaction d'un oligosaccharide dans une solution saturée de bicarbonate d'ammonium, à un pH d'environ 8 à environ 8,5, pour former un dérivé β-glycosylamine dudit oligosaccharide, l'halogénoacétylation dudit dérivé β-glycosylamine par une réaction en phase aqueuse, pour former directement le dérivé 1-N-halogénoacétamido dudit dérivé β-glycosylamine sans cristallisation sélective dans un solvant organique, la transformation dudit dérivé 1-N-halogénoacétamido en un dérivé intermédiaire 1-N-glycyl-β-glycosylamine par ammoniolyse, la puis réaction dudit dérivé intermédiaire 1-N-glycyl-β-glycosylamine avec un substrat capable de former un glycoconjugué, à liaison par la 1-N-glycyl-β-glycosylamine, dudit substrat et dudit dérivé 1-N-glycyl-β-glycosylamine.

2. Procédé selon la revendication 1, dans lequel le pH est d'environ 8,3.

3. Procédé selon la revendication 1 ou 2, dans lequel ledit dérivé halogénoacétylé est un dérivé chloroacétylé.

4. Procédé selon la revendication 3, dans lequel on utilise l'anhydride chloracétique pour former le dérivé glycosylamine halogénoacétylé.

5. Procédé selon la revendication 4, dans lequel on utilise un excès au moins 5 fois molaire d'anhydride chloracétique pour former le dérivé chloroacétylé.

6. Procédé selon la revendication 3, dans lequel on prépare le dérivé halogénoacétylé en faisant réagir ledit dérivé glycosylamine dudit oligosaccharide avec ICH₂COONHS.

7. Procédé selon l'une quelconque des revendications 1 à 6, dans lequel on fait réagir la glycyl-glycosylamine avec un fluorophore pour former un glycoconjugué à liaison par l'azote N, pratiquement dans la configuration β-anomère.

8. Procédé selon la revendication 7, dans lequel le fluorophore est un dérivé de la fluorescéine.
